(19) Europäisches Patentamt

European Patent Office

Office européen des brevets

(11) **EP 0 589 251 B1**

(12) **EUROPEAN PATENT SPECIFICATION**

(45) Date of publication and mention
of the grant of the patent:
**02.11.2000 Bulletin 2000/44**

(51) Int. Cl.$^7$: **A61N 1/39**

(21) Application number: **93113990.1**

(22) Date of filing: **01.09.1993**

(54) **Apparatus for generating multiphasic defibrillation waveforms based on pulse width ratios**

Gerät zur Erzeugung von mehrphasigen Entflimmerungswellen auf der Basis von
Impulsbreitenverhältnissen

Appareil de génération d'ondes de défibrillation polyphasées, basés sur le rapport des largeurs
d'impulsions

(84) Designated Contracting States:
**AT BE CH DE DK ES FR GB GR IE IT LI LU MC NL
PT SE**

(30) Priority: **25.09.1992 US 951252**

(43) Date of publication of application:
**30.03.1994 Bulletin 1994/13**

(73) Proprietor:
**CARDIAC PACEMAKERS, INC.
St. Paul, Minnesota 55112-5798 (US)**

(72) Inventors:
• **Dreher, Robert
Roseville, Minnesota 55113 (US)**

• **Kelly, David W.
Lino Lakes, Minnesota 55014 (US)**
• **Kraetz, Janis
Plymouth, Minnesota 55446 (US)**

(74) Representative:
**Zinnecker, Armin, Dipl.-Ing. et al
Lorenz-Seidler-Gossel,
Widenmayerstrasse 23
80538 München (DE)**

(56) References cited:
**EP-A- 0 491 649      US-A- 4 800 883
US-A- 4 821 723      US-A- 4 850 357
US-A- 4 998 531**

**EP 0 589 251 B1**

**Description**

BACKGROUND OF THE INVENTION

The present invention relates to cardioversion/defibrillation systems and more particularly to an apparatus for generating multiphasic defibrillation waveform.

Techniques for defibrillation/cardioversion have evolved over the years from a truncated exponentially decaying waveform of a capacitor to more sophisticated waveforms, such as multiphasic waveforms. In this regard, it has been found that multiphasic waveforms often are more effective in defibrillating the heart.

There are several methods known for generating multiphasic defibrillation waveforms. One method is disclosed in U.S. Patent No. 4,800,883 to Winstrom and in U.S. Patent No. 4,821,723 to Baker, Jr. et al. These patents disclose multiple capacitor networks for generating multiphasic waveforms according to a fixed-duration format, whereby the duration of each pulse is fixed but the tilt of each pulse varies with patient system impedance.

Commonly assigned U.S. Patent No. 4, 850,357 to Bach discloses a biphasic pulse generator providing a fixed tilt waveform which automatically compensates for changes in lead impedance. However, the apparatus disclosed in this patent detects several voltage levels in order to deliver the required pulse shape. Consequently, circuitry is required to detect the voltage levels.

European Patent Application EP-A-0491649 to Pless relates to an apparatus for producing configurable biphasic defibrillation waveforms. However, the apparatus is limited in its configurability of waveforms.

While these prior systems generally are effective, there is room for improvement.


SUMMARY OF THE INVENTION

Accordingly, it is an object of the present invention to provide an apparatus for generating a multiphasic defibrillation/cardioversion waveform in which the complexity of the circuitry is reduced.

It is another object of the present invention to provide an apparatus for generating a multiphasic defibrillation/cardioversion waveform in which parameters for defining phases of the waveshape are programmable.

It is still another object of the present invention to provide an apparatus for generating a multiphasic defibrillation/cardioversion waveform capable of producing the waveform with minimal voltage measurements.

Briefly, the present invention is directed to an and apparatus for generating a multiphasic defibrillation/cardioversion waveform by specifying only the initial voltage and end voltage of the first phase and the percentage of pulse widths for each subsequent phase relative to the pulse width of the first phase. Therefore, only two voltage measurements need to be made for the first phase, while for the remaining phases, only the time duration (pulse width) is detected.

The above and other objects and advantages will become more readily apparent when reference is made to the following description taken in conjunction with the accompanying drawings.


BRIEF DESCRIPTION OF THE DRAWINGS

Figure 1 is a graphical representation of a multiphasic defibrillation waveform which is generated by the apparatus according to the present invention.

Figure 2 is a flow chart illustrating the steps of generating a multiphasic defibrillation/cardioversion waveform according to the present invention.

Figure 3 is a schematic diagram of circuitry for generating a multiphasic defibrillation waveform according to the present invention.

Figure 4 is a flow chart illustrating the operation of the circuitry shown in Figure 3.


DETAILED DESCRIPTION OF THE DRAWINGS

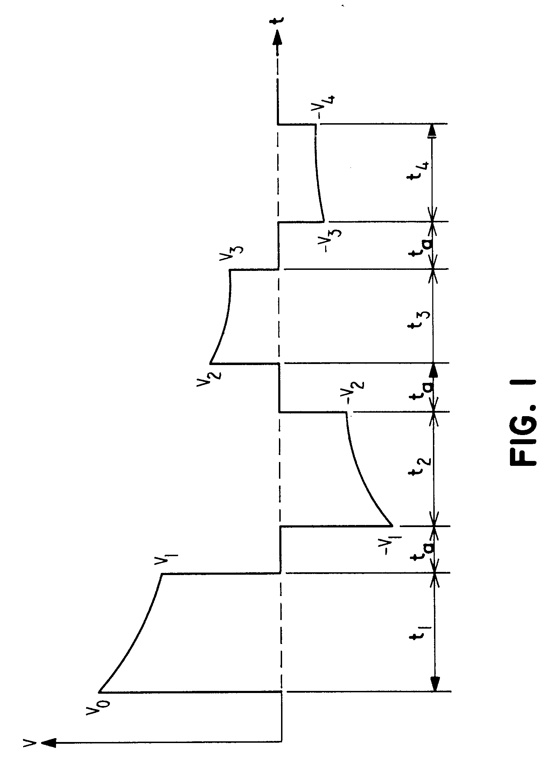

Referring first to Figure 1, a multiphasic defibrillation waveform is shown comprising four phases. According to the present invention the waveform shown in Figure 1 is generated by specifying the initial voltage $V_0$, the first truncate voltage $V_1$ and the pulse width of each of the phases 2 through 4 as a percentage of the pulse width of phase 1. Consequently, only two voltage measurements must be performed by the waveform generating circuitry and over a small voltage range ($V_0$ to $V_1$). Moreover, the time measurements may be accomplished more reliably and accurately for the higher order phases (phases 2-4) using much simpler hardware.

In general, for an n-phasic waveform (where n is any positive number), phase 1 would be specified in terms of the initial voltage $V_0$ and the truncate voltage $V_1$, and the higher order phases ($n \geq 2$) are specified as a percentage of $t_1$ where $t_1$ is the time duration (also referred to as pulse width) of the first phase.

The following is an explanation of the underlying theory of the present invention to illustrate how the method and apparatus according to the present invention is equivalent to specifying all of the cutoff (truncated) voltages for all phases. A legend for the discussion follows.

$V_0$ = initial voltage of the capacitor
$V_1$ = truncate (end) voltage of phase 1
$V_n$ = truncate voltage of phase n
$t_1$ = time duration of phase 1
$t_n$ = time duration of phase n
$P_2 = t_2/t_1$, time phase 2: time phase 1
$P_n = t_n/t_1$, time phase n: time phase 1

The truncate voltage of the first phase is found according to the discharge of a capacitor:

$V_1 = V_0 e^{-t1/RC}$, where R and C are values of the discharge resistance and capacitor. The time duration of phase 1 is, then:
$t_1 = -RC\ln(V_1/V_0)$, where ln is the natural logarithm.

According to the ratio or percentage relationship between the time duration of the phases, $t_2 = P_2 t_1 = -RC\ln(V_2/V_1)$. Consequently, substituting the expression for t1 it follows that,

$$P_2 = \frac{\ln(V_2/V_1)}{\ln(V_1/V_0)}.$$

Therefore, the truncate voltage of phase 2 is, $V_2 = (V_1/V_0)^{P2}V_1$. In general, the percentage for phase n and truncate voltage for phase n is:

$$P_n = \frac{\ln(V_n/V_{n-1})}{\ln(V_1/V_0)}$$

$$V_n = (V_1/V_0)^{Ph}V_{n-1} = (V_1/V_0)^{P2+P3+...+Pn-1+Pn}.$$

Thus, the cutoff or truncate voltage of each phase is directly related to a percentage of the pulse width of the initial phase and is independent of the actual time and RC time constant. With the knowledge of the initial and final voltages and the capacitance, the total energy delivered by the multiphase waveform can be calculated. The values of $P_1,..., P_n$ could also be calculated from percentage tilt (T), where

$$T_n = 1 - V_n/V_0.$$

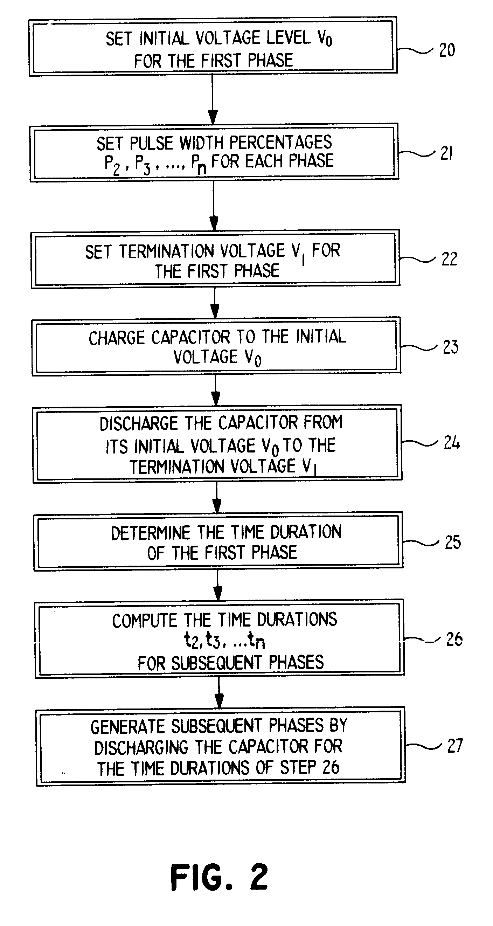

With reference to Figure 2, a method using the foregoing theory, is illustrated for generating the multiphasic waveform of Figure 1 without having to detect voltages for each phase of the waveform.

In step 20, the initial voltage level $V_0$ of the first phase is set, which voltage level also corresponds to the initial voltage level of the capacitor.

Next, in step 21, the pulse width percentages $P_2$, $P_3$, ..., $P_n$ are set for each phase, each pulse width percentage $P_2$, $P_3$, ..., $P_n$ being a ratio of the duration of the respective phase to the time duration $t_1$ of the first phase of the multiphasic waveform. As noted in the foregoing theoretical discussion, each of the pulse width percentages $P_2$, $P_3$, ..., $P_n$ can be calculated from the desired cutoff or truncate voltage of each phase, knowing the initial voltage and the termination voltage of the first phase.

In step 22, the termination voltage $V_1$ is set for the first phase of the multiphasic waveform.

In step 23, the capacitor is charged to the initial voltage $V_0$ of the first phase.

In step 24, the capacitor is discharged from its initial voltage $V_0$ to the termination voltage $V_1$ hence providing the first phase of the multiphasic waveform.

In step 25, the time duration of the first phase is determined.

In step 26, the time durations $t2_2$ $t_3$, ..., $t_n$ for subsequent phases are computed by multiplying each of the pulse width percentages $P_2$, $P_3$, ..., $P_n$ by the time duration $t_1$ of the first phase.

Finally, in step 27, subsequent phases of the multiphasic waveform are generated by discharging the capacitor for the time durations computed in step 26. Specifically, the phases are generated with alternating polarity, for example, positive polarity for odd values of n and negative polarity for even values of n.

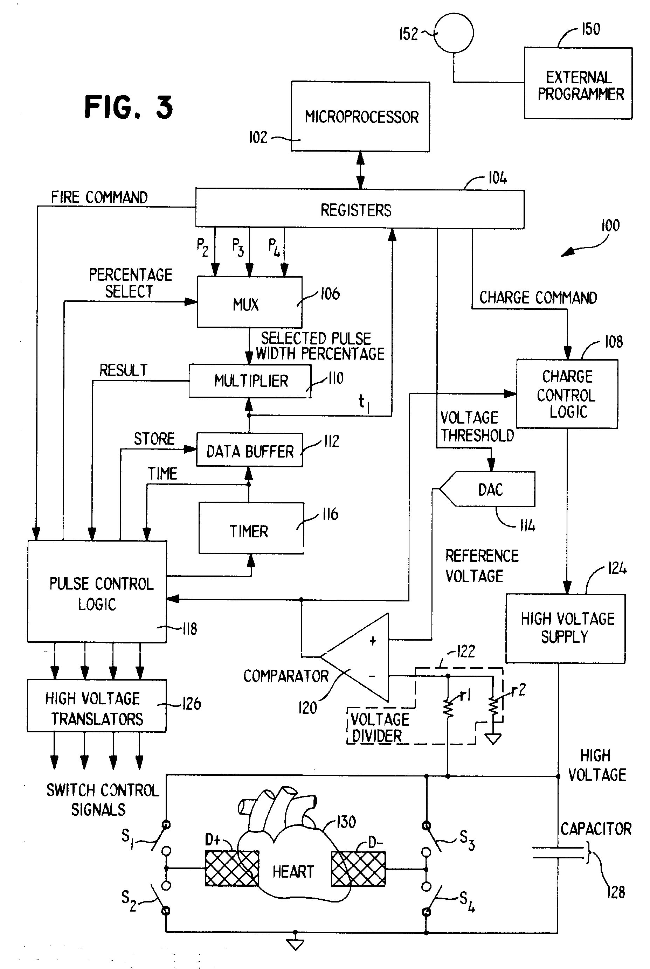

Figure 3 illustrates electrical circuitry for generating a multiphasic defibrillation waveform according to the present invention. The circuitry 100 comprises a microprocessor 102; a set of addressable registers 104 connected to the microprocessor 102; a multiplexer (MUX) 106; charge control logic 108; a multiplier 110; a data buffer 112; a digital to analog converter (DAC) 114; a timer 116; pulse control logic 118; a comparator 120; a voltage divider 122; a high voltage supply 124; high voltage translators 126; a 125 to 150 microfarad capacitor 128; four switches S1, S2, S3, and S4; and two defibrillation electrodes D+ and D- located in close proximity to a patient's heart 130.

The circuitry 100 is primarily controlled by the microprocessor 102 which itself can be either directly preprogrammed by a physician with values for $V_0$, $V_1$, $P_2$, $P_3$, ..., $P_n$ or indirectly preprogrammed using other parameters provided by the physician through means well known in the art. The physician, for example, could specify percentage tilt, voltages, and/or pulse width percentages, while an external module takes these values and calculates the proper values for $V_0$, $V_1$, $P_2$, $P_3$, ..., $P_n$ on behalf of the microprocessor 102. This data is then fed to the microprocessor 102, which either causes a defibrillation shock to be delivered immediately or causes one to be delivered automatically in response to a detected cardiac arrhythmia. A preferred microprocessor 102 is the model 68HC11 manufactured by Motorola.

The set of addressable registers 104 is connected to the microprocessor 102 and comprises a voltage threshold register for storing the values $V_0$ and $V_1$; a pulse width percentage register for storing each of the values $P_2$, $P_3$, ..., $P_n$; a command register for storing a "charge command" bit code or a "fire command" bit code; and a register for storing $t_1$. The microprocessor 102 by first storing the values for either $V_0$ or $V_1$, along with $P_2$, $P_3$, ..., $P_n$ in the set of addressable registers 104, and then selectively storing either the "fire command" bit code or the "charge command" bit code in the command register, is able to cause either delivery of a desired multiphasic defibrillation shock or the charging of the capacitor 128 to the desired peak voltage $V_0$ of the first phase, respectively.

The digital to analog converter (DAC) 114 is responsive to the value of $V_0$ or $V_1$ whichever is digitally stored in the voltage threshold register. The primary function of the DAC 114 is to take this digital value for $V_0$ or $V_1$, and in accordance therewith, provide an output reference voltage proportional to the desired peak value $V_0$ or termination value $V_1$ of the first phase. This output reference voltage is within the input range of, and is fed into, the positive input of the comparator 120.

The charge control logic 108 is responsive to the output from the comparator 120 and the bit code contained in the command register, so as to selectively enable the high voltage supply 124. By selectively enabling the high voltage supply 124 in response to detecting a "charge command" bit code in the command register, the charge control logic 108 is able to cause charging of the capacitor 128 to the peak voltage $V_0$ of the first phase. The high voltage supply itself is well known to the art. The charge control logic 108 is a sequencer which powers up the analog circuit and then charges the capacitor 128 until the comparator 120 switches.

The voltage divider 122 comprises two series-connected resistors r1 and r2 arranged such that the voltage present across the capacitor 128 is also applied across the series-connected resistors r1 and r2. In practice, the resistors have resistance values ranging from 9.7M to 10.7M ohms for r1, and 15K to 25K ohms for r2. More specifically though, the value of each resistor r1 and r2 is chosen such that the voltage at the center tap (the voltage across r2 at a ratio of 480 plus or minus 2 percent) is at a level within the input range of the comparator 120. This center tap voltage is then fed into the negative input of the comparator 120, and as a result, the negative input of the comparator 120 receives a voltage proportional to, but smaller than, that which is present across the capacitor 128.

The timer 116 is connected and responsive to the pulse control logic 118 such that, upon commencement of the first phase, the timer 116 begins counting elapsed time. The timer's output is connected to the data buffer 112 and is thereby able to provide the data buffer 112 with a measure of the time elapsed after application of the first phase. This measure of elapsed time is also provided to the pulse control logic 118.

The data buffer 112 is connected to the pulse control logic 118 via a "store" signal, and is responsive to the "store" signal such that upon the signal being applied, the data buffer 112 stores the current value of the timer's output. Since the "store" signal is applied, as will be discussed hereinafter, upon termination of the first phase, the stored value corresponds to the pulse width $t_1$ of the first phase.

The multiplexer (MUX) 106 is responsive to a "percentage select" signal from the pulse control logic 118 and, in accordance therewith, selects one of the pulse width percentage values $P_2$, $P_3$, ..., or $P_n$ from the corresponding register. The selected pulse width percentage $P_2$, $P_3$, ..., or $P_n$ is then supplied to the multiplier 110.

The multiplier 110 is connected to both the data buffer 102 and the multiplexer 106. As a result, the multiplier 110 receives both the selected pulse width percentage value $P_2$, $P_3$, ..., or $P_n$ from the multiplexer 106 and the stored value corresponding to the pulse width t1 $^\circ$f the first phase. Both of these values are multiplied by the multiplier 110 with the result being provided to the pulse control logic 118.

The pulse control logic 118 is connected and responsive to the command register, the result from the multiplier 110, the elapsed time measured by the timer 116, and the output from the comparator 120. In addition, the pulse control logic 118 provides the "percentage select" signal to the multiplexer 106, the "store" signal to the data buffer 112, and four low voltage control signals to the high voltage translators 126. The pulse control logic 118 is a digital logic sequencer that controls the pulse generator circuitry 106, 110, 112, 116 and 126.

The high voltage translators 126 are connected and receive the four low voltage control signals from the pulse control logic 118. In response to the low voltage control signals, the high voltage translators 126 produce four switch control signals corresponding to the low voltage control signals, but of higher voltage. In this regard, the translators 126 simply step-up the voltage of the low voltage control signals.

Although the actual connections are not shown in Figure 3, each of the switch control signals is connected to one of the switches S1, S2, S3, and S4. The switches S1, S2, S3, and S4 are arranged and responsive to the switch control signals so as to selectively make or break a series electrical circuit through the heart 130, the capacitor 128, and the defibrillation electrodes D+ and D-. Depending on which switches are closed, the circuitry 100 either provides the heart 130 with a positive polarity voltage (where D- is connected to ground) or a negative polarity voltage (where D+ is connected to ground). As can be seen from Figure 3, closing switches s1 and s4 provides the heart 130 with a positive polarity voltage, while closing switches s2 and s3 provides a negative polarity voltage.

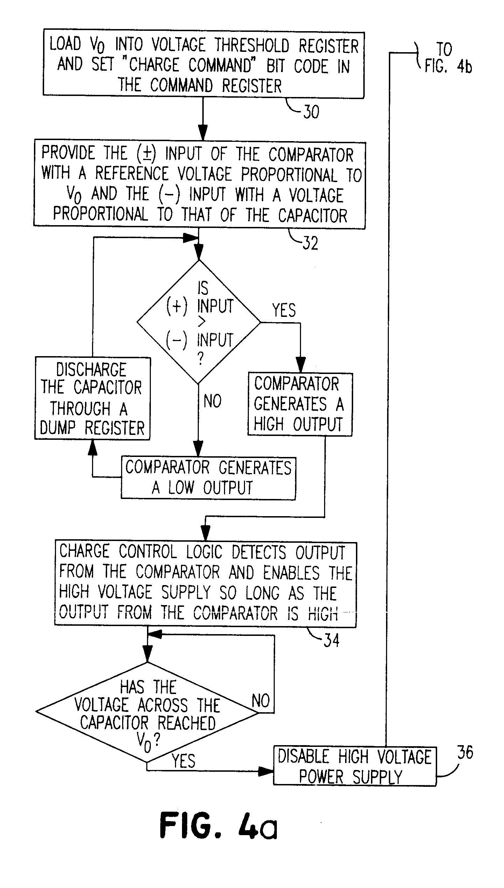

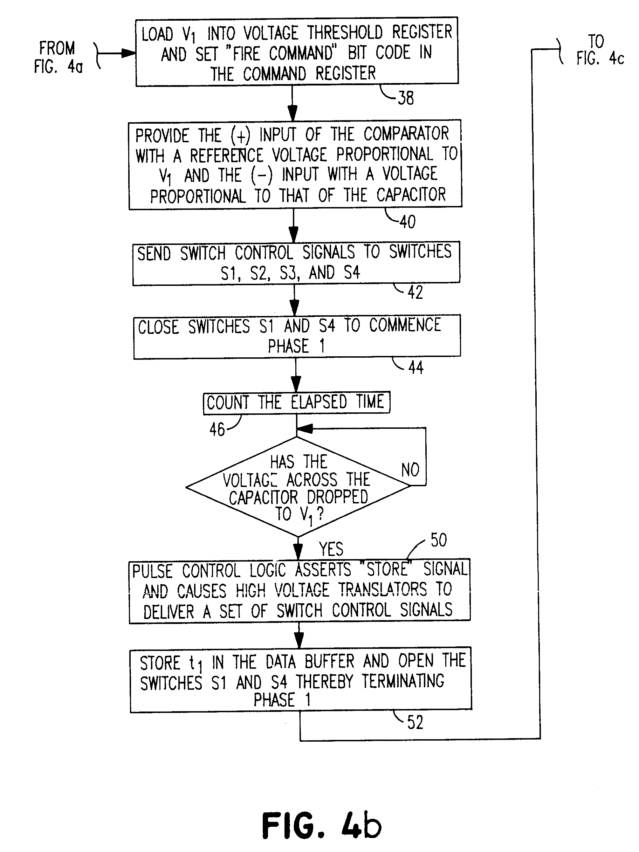

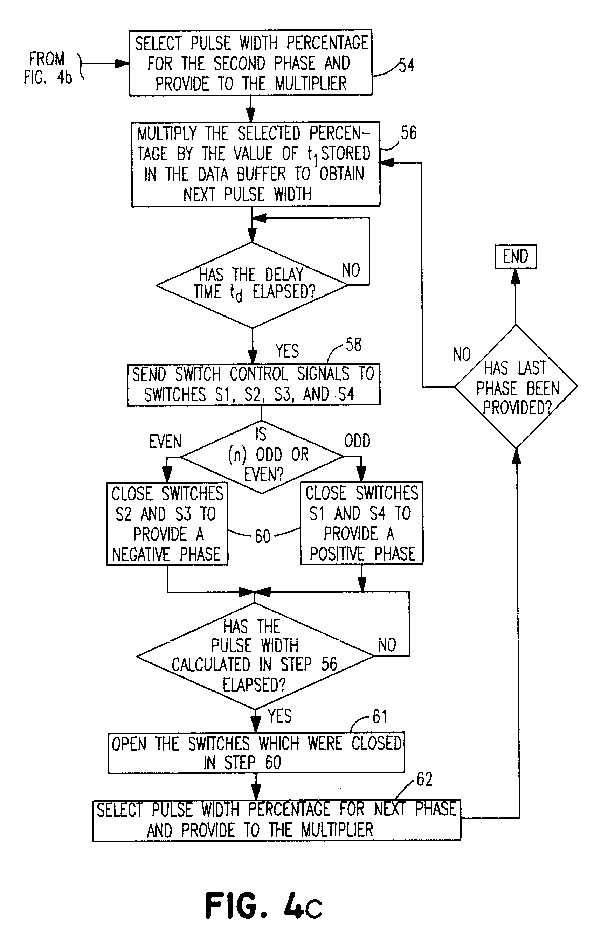

With reference to Figure 4, operation of the circuitry 100 in delivering the multiphasic defibrillation shock of Figure 1, will now be described. In step 30, the microprocessor 102 loads the peak voltage value $V_0$ into the voltage threshold register, and sets the "charge command" bit code into the command register. The actual value of $V_0$ depends on several external factors including electrode configuration, the patient's defibrillation threshold, patient and electrode impedances, etc. Nevertheless, typical values for $V_0$ range from 45 to 715 volts.

In step 32, the DAC 114 detects the value $V_0$ and, in response thereto, creates the output reference voltage proportional to the desired peak voltage value $V_0$ of the first phase, the reference voltage being provided to the positive input of the comparator 120. Meanwhile, the voltage divider 122 divides the voltage present on the capacitor 128 to a level within the input range of the comparator 120, and provides this lower voltage to the negative input of the comparator 120.

In step 34, the charge control logic 108 detects the output from the comparator 120, and in response to this output selectively enables the high voltage supply 124. More specifically, when the voltage across the capacitor 128 is less than $V_0$, the input to the negative terminal of the comparator 120 is less than that of the positive terminal. This, in turn, causes a high output on the comparator 120. The charge control logic 108 responds to this high output by enabling the high voltage supply 124 which begins charging the capacitor 128. If, on the other hand, the comparator's output begins low, then this may be an indication that the capacitor 128 is over-charged. Accordingly, the capacitor 128 is discharged through a dump resistor (not shown), which can be of any design generally known in the art. Examples of such dump resistors are shown in U.S. Patents Nos. 4,316,472 and 4,488,555 to Imran and Mirowski, respectively.

In step 36, when the voltage on the capacitor 128 reaches $V_0$ while being charged, the comparator's output goes from high to low, signalling the charge control logic 108 to disable the high voltage supply 124. A signal from the charge control logic 108 is provided to the microprocessor 102 when the capacitor 128 is fully charged.

Next, in step 38, the microprocessor 102 loads the first phase termination voltage value $V_1$ into the voltage threshold register and sets the "fire command" bit code in the command register. The actual value for $V_1$, like $V_0$, depends upon several external factors, but nevertheless may range between 18 and 286 volts.

In step 40, the DAC 114 detects the digitally stored value for $V_1$ and, in response thereto, creates a reference voltage proportional to the termination voltage $V_1$ of the first phase, the reference voltage being provided to the positive input of the comparator 120. In the meantime, the voltage divider 122 continues to divide the voltage present on the capacitor 128 to a level within the input range of the comparator 120, and provides this lower voltage to the negative input of the comparator 120.

In step 42, upon detecting the "fire command" bit code in the command register, the pulse control logic 118 sends appropriate control signals to the switches S1, S2, S3, and S4 through the high voltage translators 126, which signals are output by the translators 126 as higher voltage switch control signals.

Next, in step 44, the switch control signals cause the switches S1 and S4 to close, thus creating a positive polarity series circuit through the capacitor 128, the heart 130, and the defibrillation electrodes D+ and D-. As a result, the capacitor 128 begins to discharge through the heart 130 thereby commencing the first phase of the multiphasic defibrillation shock.

In step 46, while the first phase is being applied to the heart 130, the timer 116 counts elapsed time and the pulse control logic 118 detects the output from the comparator 120 awaiting a transition from a low output to a high output. When the voltage on the capacitor 128 decays to the first phase termination voltage $V_1$ the comparator 120 output goes from low to high, thereby signalling the pulse control logic 118 to open the switches S1 and S4 and terminate the first phase.

In step 50, the pulse control logic 118 detects the low to high transition in the comparator's output, and in response thereto, sends the "store" signal to the data buffer 112. The pulse control logic 118 further causes a set of switch control signals to be sent from the high voltage translators 126 to open switches S1 and S4.

In step 52, upon receiving the "store signal", the data buffer 112 stores the elapsed time corresponding to the pulse width $t_1$ of the first phase. The pulse width $t_1$ may be between 1 and 24 milliseconds long. In addition, the switches S1 and S4 are opened to prevent further discharge of the capacitor 12 8 and to thereby terminate the first phase.

Next, in step 54, the pulse control logic 118, using the "percentage select" signal, causes the multiplexer (Mm) 106 to select the second pulse width percentage $P_2$ from the corresponding register, which pulse width percentage $P_2$ is provided to the multiplier 110 for multiplication with the first phase pulse width $t_1$.

In step 56, the multiplier 110 receives the value of $P_2$ from the multiplexer 106, as well as the value of $t_1$ from the data buffer 112, and multiplies both values to obtain a result which corresponds to the pulse width $t_2$ of the second phase, this result t2 being provided to the pulse control logic 118.

In step 58, after a delay time $t_d$, the pulse control logic 118 sends appropriate control signals to the switches S1, S2, S3, and S4 through the high voltage translators 126, which signals are output by the translators 126 as higher voltage switch control signals.

In step 60, the switch control signals emitted in step 58 cause switches S2 and S3 (since the value of n is even) to close thus creating a negative polarity series circuit through the capacitor 128, the heart 130, and the defibrillation electrodes D+ and D-. As a result, the capacitor 128 again begins to discharge through the heart 130, but this time with negative polarity. The second phase of the multiphasic defibrillation shock is thereby commenced.

In step 61, at the end of the pulse width $t_2$, the pulse control logic 118 causes the switches S2 and S3 to open, thus terminating the second phase of the multiphasic shock.

In step 62, the next consecutive pulse width percentage $P_3$, $P_4$, ..., or $P_n$ is selected using the multiplexer 106, and the steps 56 - 61 are repeated for each value of $P_3$, $P_4$, ..., or $P_n$ and each value of $t_3$, ..., $t_4$, or $t_n$ or tn until the final percentage Pn has been selected and the last phase has been delivered to the heart 130 with the respective pulse width $t_n$. When step 60 is repeated for odd values of n, switches S1 and S4 are closed to deliver a positive polarity voltage to the heart 130. For even values of n, switches S2 and S3 are closed to deliver a negative polarity voltage to the heart 130. In this manner, successive phases continue to alternate polarity until the last phase has been delivered.

For defibrillation purposes, the pulse width percentages $P_2$, $P_3$, $P_4$, ..., $P_n$ preferably range in value from 25% to 150% such that the pulse widths $t_2$, $t_3$, ..., $t_n$ range in value from to 1 to 36 milliseconds. Likewise, the delay time $t_d$ ranges from 0.8 to 1.2 milliseconds.

In addition to the foregoing, it is well understood that the external programmer 150 and other similar devices can also be used to program the values of $V_0$, $V_1$, $P_2$, $P_3$, ..., $P_n$, into the microprocessor 102 of the present invention. Programming of defibrillator operating parameters using an external programmer device is well known in the art.

Furthermore, modifications may be made to the circuitry 100 shown in Figure 3 without departing from the present invention. Particularly, many functions performed by the microprocessor 102 may be performed by analog circuitry. Also the charging and discharging of the capacitor 128 may actually be accomplished using two separate circuits, one circuit for charging the capacitor 128 and one circuit for enabling and disabling the discharge of the capacitor 128.

**Claims**

1. Apparatus for generating a multiphasic waveform, said apparatus comprising:

   capacitive means (128) for storing electrical energy;

   programmable processing means (102) for controlling the charging and discharging of said capacitive means;

   at least one addressable register (104) for storing command bits codes and waveform parameters provided by said programmable processing means;

   timing means (116) for measuring the time duration of the first phase of the multphasic waveform;

   data storage means (112) responsive to said timing means for storing a value corresponding to the time duration of the first phase;

   comparison means (114, 120, 122) for comparing voltage-related waveform parameters stored in said at least one addressable register to the voltage across the capacitive means;

   multiplexing means (106) connected to said at least one register for selectively choosing a waveform parameter corresponding to a desired pulse width percentage;

multiplication means (110) responsive to the selectively chosen waveform parameter from said multiplexing means and also responsive to said timing means for multiplying the time duration of the first phase by the desired pulse width percentage and for generating a signal indicative of the result thereof;

at least two defibrillation electrodes (D+, D-) adapted to be located near a patient's heart;

switch means ($S_1$, $S_2$, $S_3$, $S_4$) for selectively discharging said capacitive means via said at least two defibrillation electrodes;

charging means (124) for charging the capacitive means; and

charge control means (108) responsive to the comparison means and responsive to said at least one register, for controlling the charging means; and

pulse control means (118) for controlling said switch means to discharge the capacitive means in accordance with voltage-related waveform parameters for the first phase and waveform parameters corresponding to pulse width percentages for subsequent phases, said pulse control means being responsive to the comparison means, the timing means, the multiplication means, and said at least one register.

2. The apparatus of claim 1, wherein said capacitive means (128) is a 125 to 150 microfarad capacitor.

3. The apparatus of claim 1 or 2 wherein said comparison means comprises a comparator (120), a voltage divider (122), and a digital-to-analog converter (114); said voltage divider providing the comparator with a first input voltage proportional to, but less than, the voltage across the capacitive means (128), and said digital-to-analog converter providing the comparator with a second input voltage proportional to the value of a voltage-related parameter currently stored in said at least one addressable register (104).

4. Apparatus for generating a time ratiometric multiphasic waveform comprising:

means for setting an initial voltage level ($V_0$) of a capacitor corresponding to an initial voltage level of a first phase of a multiphasic waveform;

means for setting each phase in the multiphasic waveform subsequent to the first phase a desired time ratio of the duration of the respecitve phase to a time duration of the first phase of the multiphasic waveform;

means for setting the end voltage level ($V_1$) of the first phase of a multiphasic waveform;

means for charging the capacitor (128) to the initial voltage level;

means for discharging the capacitor from the initial voltage level ($V_0$) to the end voltage level ($V_1$) to cause the first phase of the multiphasic waveform;

means for determining the time duration ($t_1$) of the first phase of the multiphasic waveform;

means for computing time durations ($t_2$, ..., $t_n$) of subsequent phases of the multiphasic waveform based on the time duration of the first phase and the ratios of the time durations of the subsequent phases relative to the first phase; and

means for controlling the discharge of the capacitor (128) according to the time durations of subsequent phases to generate the subsequent phases of the multiphasic waveform.

5. The apparatus of claim 4, wherein said means for computing time durations of the subsequent phases comprises means for multiplying the time duration ratio for the corresponding phase by the time duration of the first phase.

6. The apparatus of claim 4 or 5, further comprising means for alternating the polarity of successive phases of the multiphasic waveform.

7. The apparatus of any one of claims 4 to 6 further comprising:

means for setting for each phase in the multiphasic waveform subsequent to the first phase parameters for defining the shape of phases subsequent the first phase in terms relative to the shape of the first phase of the multiphasic waveform;

means for computing waveshape parameters of subsequent phases of the multiphasic waveform based on the time duration of the first phase, the parameters of the subsequent phases, the initial voltage level of the first phase and the end voltage level of the first phase; and

means for controlling the discharge of the capacitor according to the waveshape parameters of subsequent phases to generate the subsequent phases of the multiphasic waveform.

8. The apparatus of any one of claims 4 to 7, further comprising:

means for loading into a voltage threshold register, a value corresponding to a desired initial voltage of the first phase of the multiphasic waveform;

means for comparing said value corresponding to a desired initial voltage to a voltage currently across a capacitor;

means for loading a charge command bit code into a command register;

means for changing the capacitor whenever the charge command bit code is in the command register and the voltage across the capacitor is less than the value corresponding to the desired initial voltage of the first phase;

means for terminating said charging of the capacitor when the voltage across said capacitor reaches the desired initial voltage of the first phase;

means for loading into the voltage threshold register, a value corresponding to the desired termination voltage of the first phase;

means for loading a fire command bit code into the command register;

means for discharging the capacitor in response to the fire command bit code being loaded into the command register thereby commencing the first phase of the multiphasic waveform;

means for terminating said discharging of the capacitor upon said voltage across the capacitor decaying to the value most recently loaded into the voltage threshold register thereby terminating said first phase;

means for storing said timer duration of the first phase in a data buffer;

means for selecting a pulse width percentage corresponding to a next phase of the multiphasic waveform; and

means for multiplying the time duration of the first phase by said pulse width percentage to thereby determine the time duration of the next phase of the multiphasic waveform.

**Patentansprüche**

1. Vorrichtung zur Erzeugung einer mehrphasigen Wellenform, wobei die Vorrichtung Folgendes umfasst:

- kapazitives Mittel (128) zum Speichern elektrischer Energie;
- programmierbares Verarbeitungsmittel (102) zur Steuerung des Aufladens und Entladens des kapazitiven Mittels;
- mindestens ein adressierbares Register (104) zum Speichern von Befehlsbitcodes und Wellenformparametern, die von dem programmierbaren Verarbeitungsmittel geliefert werden;
- Zeiterfassungsmittel (116) zum Messen der Zeitlänge der ersten Phase der mehrphasigen Wellenform;
- Datenspeichermittel (112), das auf das Zeiterfassungsmittel reagiert, zum Speichern eines der Zeitlänge der ersten Phase entsprechenden Werts;

- Vergleichsmittel (114, 120, 122) zum Vergleichen der mit der Spannung in Zusammenhang stehenden Wellenformparameter, die in dem mindestens einen adressierbaren Register gespeichert sind, mit der Spannung über dem kapazitiven Mittel;
- mit dem mindestens einen Register verbundenes Multiplexingmittel (106) zum selektiven Auswählen eines Wellenformparameters, der einem gewünschten Pulsbreitenprozentsatz entspricht;
- Multiplikationsmittel (110), das auf den selektiv ausgewählten Wellenformparameter von dem Multiplexingmittel reagiert und auch auf das Zeiterfassungsmittel zum Multiplizieren der Zeitlänge der ersten Phase mit dem gewünschten Pulsbreitenprozentsatz und zur Erzeugung eines auf dieses Ergebnis hinweisenden Signals reagiert;
- mindestens zwei zur Positionierung nahe des Herzens eines Patienten ausgelegte Defibrillationselektroden (D+, D-);
- Umschaltmittel ($S_1$, $S_2$, $S_3$, $S_4$) zur selektiven Entladung des kapazitiven Mittels über die mindestens zwei Defibrillationselektroden;
- Auflademittel (124) zum Aufladen des kapazitiven Mittels und
- auf das Vergleichsmittel und auf das mindestens eine Register reagierendes Aufladungssteuermittel (108) zur Steuerung des Auflademittels und
- Pulssteuerungsmittel (108) zur Steuerung des Umschaltmittels, so dass das kapazitive Mittel gemäß der mit der Spannung in Zusammenhang stehenden Wellenformparameter für die erste Phase und der Wellenformparameter, die den Pulsbreitenprozentsätzen für spätere Phasen entsprechen, entladen wird, wobei das Pulssteuerungsmittel auf das Vergleichsmittel, das Zeiterfassungsmittel, das Multiplikationsmittel und das mindestens eine Register reagiert.

2. Vorrichtung nach Anspruch 1, dadurch gekennzeichnet, dass das kapazitive Mittel (128) ein 125- bis 150-Mikrofaradkondensator ist.

3. Vorrichtung nach Anspruch 1 oder 2, dadurch gekennzeichnet, dass das Vergleichsmittel einen Komparator (120), einen Spannungsteiler (122) und einen Digital-Analog-Wandler (114) umfasst, wobei der Spannungsteiler dem Komparator eine erste Eingangsspannung liefert, die proportional zu der Spannung über dem kapazitiven Mittel (128) ist, aber kleiner als diese ist, und der Digital-Analog-Wandler dem Komparator eine zweite Eingangsspannung liefert, die proportional zu dem Wert eines mit der Spannung in Zusammenhang stehenden Parameters ist, der aktuell in dem mindestens einen adressierbaren Register (104) gespeichert ist

4. Vorrichtung zur Erzeugung einer zeitratiometrischen mehrphasigen Wellenform, welche Folgendes umfasst:

- Mittel zum Einstellen eines Anfangsspannungspegels ($V_0$) eines Kondensators entsprechend einem Anfangsspannungspegel einer ersten Phase einer mehrphasigen Wellenform;
- Mittel zum Einstellen eines gewünschten Zeitverhältnisses der Dauer der jeweiligen Phase zu einer Zeitlänge der ersten Phase der mehrphasigen Wellenform für jeder Phase in der mehrphasigen Wellenform nach der ersten Phase;
- Mittel zum Einstellend des Endspannungspegels ($V_1$) der ersten Phase einer mehrphasigen Wellenform;
- Mittel zum Aufladen des Kondensators (128) auf den Anfangsspannungspegel;
- Mittel zum Entladen des Kondensators von dem Anfangsspannungspegel ($V_0$) auf den Endspannungspegel ($V_1$), um die erste Phase der mehrphasigen Wellenform hervorzurufen;
- Mittel zur Ermittlung der Zeitlänge ($t_1$) der ersten Phase der mehrphasigen Wellenform;
- Mittel zur Berechnung der Zeitlängen ($t_2$, ..., $t_n$) der folgenden Phasen der mehrphasigen Wellenform anhand der Zeitlänge der ersten Phase und den Verhältnissen der Zeitlängen der folgenden Phasen relativ zu der ersten Phase und
- Mittel zum Steuern der Entladung des Kondensators (128) gemäß den Zeitlängen der folgenden Phasen, um die folgenden Phasen der mehrphasigen Wellenform zu erzeugen.

5. Vorrichtung nach Anspruch 4, dadurch gekennzeichnet, dass das Mittel zum Berechnen der Zeitlängen der folgenden Phasen Mittel zum Multiplizieren des Zeitlängenverhältnisses für die entsprechende Phase mit der Zeitlänge der ersten Phase umfasst.

6. Vorrichtung nach Anspruch 4 oder 5, welches weiterhin Mittel zum Ändern der Polarität der folgenden Phase der mehrphasigen Wellenform umfasst.

7. Vorrichtung nach einem der Ansprüche 4 bis 6, welche weiterhin Folgendes umfasst:

   - Mittel zum Einstellen von Parametern zur Festlegung der Form der Phasen nach der ersten Phase im Verhältnis zur Form der ersten Phase der mehrphasigen Wellenform für jede Phase in der mehrphasigen Wellenform nach der ersten Phase;
   - Mittel zum Berechnen von Wellenformparametern der folgenden Phasen der mehrphasigen Wellenform anhand der Zeitlänge der ersten Phase, der Parameter der folgenden Phasen, des Anfangsspannungspegels der ersten Phase und des Endspannungspegels der ersten Phase und
   - Mittel zum Steuern der Entladung des Kondensators gemäß Wellenformparametern der folgenden Phasen, um die folgenden Phasen der mehrphasigen Wellenform zu erzeugen.

8. Vorrichtung nach einem der Ansprüche 4 bis 7, welche weiterhin Folgendes umfasst:

   - Mittel zum Laden eines Werts entsprechend einer gewünschten Anfangsspannung der ersten Phase der mehrphasigen Wellenform in ein Spannungsschwellwertregister;
   - Mittel zum Vergleichen des Werts entsprechend einer gewünschten Anfangsspannung mit einer derzeit über einem Kondensator anliegenden Spannung;
   - Mittel zum Laden eines Ladungsbefehlsbitcodes in ein Befehlsregister;
   - Mittel zum Ändern des Kondensators, sobald der Ladungsbefehlsbitcode sich in dem Befehlsregister befindet und die Spannung über dem Kondensator geringer als der Wert entsprechend der gewünschten Anfangsspannung der ersten Phase ist;
   - Mittel zum Beenden des Aufladens des Kondensators, wenn die Spannung über dem Kondensator die gewünschte Anfangsspannung der ersten Phase erreicht;
   - Mittel zum Laden eines Werts entsprechend der gewünschten Beendigungsspannung der ersten Phase in das Spannungsschwellwertregister;
   - Mittel zum Laden eines Feuerbefehlsbitcodes in das Befehlsregister;
   - Mittel zum Entladen des Kondensators in Reaktion auf den Feuerbefehlsbitcode, der gerade in das Befehlsregister geladen wird, um damit die erste Phase der mehrphasigen Wellenform einzuleiten;
   - Mittel zum Beenden der Entladung des Kondensators, sobald die Spannung über dem Kondensator auf den zuletzt in das Spannungsschwellwertregister geladenen Wert abnimmt, wodurch die erste Phase beendet wird;
   - Mittel zum Speichern der Zeitlänge der ersten Phase in einem Datenpuffer;
   - Mittel zum Wählen eines Pulsbreitenprozentsatzes entsprechend einer nächsten Phase der mehrphasigen Wellenform und
   - Mittel zum Multiplizieren der Zeitlänge der ersten Phase mit dem Pulsbreitenprozentsatz, um somit die Zeitlänge der nächsten Phase der mehrphasigen Wellenform zu ermitteln.

**Revendications**

1. Appareil de génération d'une forma d'onde polyphasée, ledit appareil comprenant :

   des moyens capacitifs (128) pour stocker de l'énergie électrique ;

   des moyens de traitement programmables (102) pour commander le chargement et le déchargement desdits moyens capacitifs ;

   au moins un registre adressable (104) pour stocker des codes de bits d'ordre et des paramètres de forme d'onde fournis par lesdits moyens de traitement programmables ;

   des moyens de synchronisation (116) pour mesurer la durée de la première phase de la forme d'onde polyphasée

   des moyens de stockage de données (112) répondant auxdits moyens de synchronisation pour stocker une valeur correspondant à la durée de la première phase ;

   des moyens de comparaison (114, 120, 122) pour comparer des paramètres de forme d'onde reliés à la tension qui sont stockés dans cet au moins un registre adressable à la tension aux bornes des moyens capacitif ;

des moyens de multiplixage (106) reliés à cet au moins un registre pour choisir de manière sélective un paramètre de forme d'onde correspondant à un pourcentage de largeur d'impulsion désiré ;

des moyens de multiplication (110) répondant au paramètre de forme d'onde choisi de manière sélective à partir desdits moyens de multiplixage et répondant également aux moyens de synchronisation pour multiplier la durée de la première phase par le pourcentage de largeur d'impulsion désirée et pour former un signal indicateur de son résultat ;

au moins deux électrodes de défibrillation (D+, D-) prévues tour être placées près du coeur d'un patient ;

des moyens de commutation ($S_1$, $S_2$, $S_3$, $S_4$) pour décharger de manière sélective lesdits moyens capacitifs par l'intermédiaire de ces au moins deux électrodes de défibrillation ;

des moyens de charge (124) pour charger les moyens capacitifs; et

des moyens de commande de charge (108) répondant aux moyens de comparaison et répondant à cet au moins un registre, pour commander les moyens de chargement; et

des moyens de commande d'impulsion (118) pour commander lesdits moyens de commutation afin de décharger les moyens capacitifs en accord avec des paramètres de forme d'onde reliés à la tension pour la première phase et avec des paramètres de forme d'onde correspondant à des pourcentages de largeur d'impulsion pour des phases ultérieures, lesdits moyens de commande d'impulsion répondant aux moyens de comparaison, aux moyens de synchronisation, aux moyens de multiplication, et à cet au moins un registre.

2. L'appareil de la revendication 1, dans lequel lesdits moyens capacitifs (128) sont constitués par un condensateur de 125 à 150 microfarads.

3. L'appareil de la revendication 1 ou 2 dans lequel lesdits moyens de comparaison comprennent un comparateur (120), un diviseur de tension (122), et un convertisseur numérique-analogique (114) ; ledit diviseur de tension appliquant au comparateur une première tension d'entrée proportionnelle mais inférieure à la tension aux bornes des moyens capacitifs (128), et ledit convertisseur numérique-analogique appliquant au comparateur une seconde tension d'entrée proportionnelle à la valeur d'un paramètre relié à la tension actuellement stocké dans cet au moins un registre adressable (104).

4. Appareil de génération d'une forme d'onde polyphasée logométrique temporelle comprenant :

des moyens pour régler un niveau de tension initial ($V_0$) d'un condensateur correspondant à un niveau de tension initiai d'une première phase d'une forme d'onde polyphasée ;

des moyens pour régler chaque phase de la forme d'onde polyphasée ultérieure la première phase à un rapport temporel désiré de la durée de la phase respective à une durée de la première phase de la forme d'onde polyphasée ;

des moyens pour régler le niveau de tension final ($V_1$) de la première phase d'une forme d'onde polyphasée ;

des moyens pour charger le condensateur (128) au niveau de tension initial ;

des moyens pour décharger le condensateur depuis le niveau de tension initial ($V_0$) jusqu'au niveau de tension final ($V_1$) pour amener la première phase de la forme d'onde polyphasée ;

des moyens pour déterminer la durée ($t_1$) de la première phase de la forme d'onde polyphasée ;

des moyens pour calculer des durées ($t_2$, ..., $t_n$) des phases ultérieures de la forme d'onde polyphasée sur la base de la durée de la première phase et des rapports des durées des phases ultérieures par rapport à la première phase ; et

des moyens pour commander la décharge du condensateur (128) conformément aux durées de phases ultérieures pour produire les phases ultérieures de la forme d'onde polyphasée ;

**5.** L'appareil de la revendication 4, dans lequel lesdits moyens pour calculer les durées des phases ultérieures comprennent des moyens pour multiplier le rapport de durée pour la phase correspondante par la durée de la première phase.

**6.** L'appareil de la revendication 4 ou 5, comprenant en outre des moyens pour alterner la polarité de phase successive de la forme d'onde polyphasée.

**7.** L'appareil d'une quelconque des revendications 4 à 6 comprenant en outre :

des moyens pour régler, pour chaque phase de la forme d'onde polyphasée ultérieure à la première phase, des paramètres pour définir la forme de phases ultérieures à la première phase en termes relatifs à la forme de la première phase de la forme d'onde polyphasée ;

des moyens pour calculer des paramètres de forme d'onde de phases ultérieures de la forme d'onde polyphasée sur la base de la durée de la première phase, des paramètres

des phases ultérieures, du niveau de tension initial de la première phase et du niveau de tension final de la première phase ; et

des moyens pour commander la décharge du condensateur selon les paramètres de forme d'onde de phases ultérieures afin de produire les phases ultérieures de la forme d'onde polyphasée ;

**8.** L'appareil d'une quelconque des revendications 4 à 7, comprenant en outre :

des moyens pour charger, dans un registre de seuil de tension, une valeur correspondant à une tension initiale désirée de la première phase de la forme d'onde polyphasée ;

des moyens pour comparer, à une tension actuellement aux barbes d'un condensateur, ladite valeur correspondant à la tension initiale désirée ;

des moyens pour charger un code de bits d'ordre de charge dans un registre d'ordre ;

des moyens pour charger le condensateur à chaque fois que le code de bits d'ordre de charge se trouve dans le registre d'ordre et que la tension aux bornes du condensateur est inférieure à la valeur correspondant à la valeur initiale désirée de la première phase ;

des moyens pour terminer ladite charge du condensateur lorsque la tension aux bornes dudit condensateur atteint la tension initiale désirée de la première phase ;

des moyens pour charger, dans le registre de seuil de tension, une valeur correspondant à la tension de terminaison désirée de la première phase ;

des moyens pour charger un code de bits d'ordre d'amorçage dans le registre d'ordre ;

des moyens pour décharger le condensateur en réponse au fait que le code de bits d'ordre d'amorçage est chargé dans le registre d'ordre en commençant ainsi la première phase de la forme d'onde polyphasée ;

des moyens pour terminer ladite décharge du condensateur lorsque la tension aux bornes du condensateur décroît à la valeur la plus récemment chargée dans le registre de seuil de tension en terminant ainsi ladite première phase ;

des moyens pour stocker ladite durée de la première phase dans un tampon de données ;

des moyens pour sélectionner un pourcentage de largeur d'impulsion correspondant à une phase suivante de la forme d'onde polyphasée ; et

des moyens pour multiplier la durée de la première phase par ledit pourcentage de largeur d'impulsion de manière ainsi à déterminer la durée de la phase suivante de la forme d'onde polyphasée.

FIG. I

```
┌─────────────────────────────────┐
│   SET INITIAL VOLTAGE LEVEL V₀   │──── 20
│      FOR THE FIRST PHASE         │
└─────────────────────────────────┘
                │
                ▼
┌─────────────────────────────────┐
│   SET PULSE WIDTH PERCENTAGES    │──── 21
│  P₂ , P₃ , ..., Pₙ FOR EACH PHASE │
└─────────────────────────────────┘
                │
                ▼
┌─────────────────────────────────┐
│  SET TERMINATION VOLTAGE Vₗ FOR  │──── 22
│        THE FIRST PHASE           │
└─────────────────────────────────┘
                │
                ▼
┌─────────────────────────────────┐
│   CHARGE CAPACITOR TO THE INITIAL │──── 23
│         VOLTAGE V₀               │
└─────────────────────────────────┘
                │
                ▼
┌─────────────────────────────────┐
│  DISCHARGE THE CAPACITOR FROM     │──── 24
│  ITS INITIAL VOLTAGE V₀ TO THE    │
│   TERMINATION VOLTAGE Vₗ          │
└─────────────────────────────────┘
                │
                ▼
┌─────────────────────────────────┐
│   DETERMINE THE TIME DURATION     │──── 25
│      OF THE FIRST PHASE          │
└─────────────────────────────────┘
                │
                ▼
┌─────────────────────────────────┐
│   COMPUTE THE TIME DURATIONS      │──── 26
│        t₂, t₃, ...tₙ             │
│     FOR SUBSEQUENT PHASES        │
└─────────────────────────────────┘
                │
                ▼
┌─────────────────────────────────┐
│  GENERATE SUBSEQUENT PHASES BY    │──── 27
│  DISCHARGING THE CAPACITOR FOR    │
│   THE TIME DURATIONS OF STEP 26  │
└─────────────────────────────────┘
```

# FIG. 2

## FIG. 3

LOAD $V_0$ INTO VOLTAGE THRESHOLD REGISTER
AND SET "CHARGE COMMAND" BIT CODE IN
THE COMMAND REGISTER
⌐30

TO
FIG. 4b

PROVIDE THE (±) INPUT OF THE COMPARATOR
WITH A REFERENCE VOLTAGE PROPORTIONAL TO
$V_0$ AND THE (−) INPUT WITH A VOLTAGE
PROPORTIONAL TO THAT OF THE CAPACITOR
⌐32

IS
(+) INPUT
>
(−) INPUT
?

YES

NO

DISCHARGE
THE CAPACITOR
THROUGH A
DUMP REGISTER

COMPARATOR
GENERATES A
HIGH OUTPUT

COMPARATOR GENERATES
A LOW OUTPUT

CHARGE CONTROL LOGIC DETECTS OUTPUT
FROM THE COMPARATOR AND ENABLES THE
HIGH VOLTAGE SUPPLY SO LONG AS THE
OUTPUT FROM THE COMPARATOR IS HIGH
⌐34

HAS THE
VOLTAGE ACROSS THE
CAPACITOR REACHED
$V_0$?

NO

YES

DISABLE HIGH VOLTAGE
POWER SUPPLY
36

# FIG. 4a

FROM
FIG. 4a

LOAD $V_1$ INTO VOLTAGE THRESHOLD REGISTER
AND SET "FIRE COMMAND" BIT CODE IN
THE COMMAND REGISTER

⌐38

TO
FIG. 4c

PROVIDE THE (+) INPUT OF THE COMPARATOR
WITH A REFERENCE VOLTAGE PROPORTIONAL TO
$V_1$ AND THE (−) INPUT WITH A VOLTAGE
PROPORTIONAL TO THAT OF THE CAPACITOR

⌐40

SEND SWITCH CONTROL SIGNALS TO SWITCHES
S1, S2, S3, AND S4

⌐42

CLOSE SWITCHES S1 AND S4 TO COMMENCE
PHASE 1

⌐44

COUNT THE ELAPSED TIME

46⌐

HAS THE
VOLTAGE ACROSS THE
CAPACITOR DROPPED
TO $V_1$?

NO

YES

⌐50

PULSE CONTROL LOGIC ASSERTS "STORE" SIGNAL
AND CAUSES HIGH VOLTAGE TRANSLATORS TO
DELIVER A SET OF SWITCH CONTROL SIGNALS

STORE $t_1$ IN THE DATA BUFFER AND OPEN THE
SWITCHES S1 AND S4 THEREBY TERMINATING
PHASE 1

⌐52

# FIG. 4b

FROM
FIG. 4b

SELECT PULSE WIDTH PERCENTAGE
FOR THE SECOND PHASE AND
PROVIDE TO THE MULTIPLIER                    54

MULTIPLY THE SELECTED PERCEN-
TAGE BY THE VALUE OF $t_1$ STORED            56
IN THE DATA BUFFER TO OBTAIN
NEXT PULSE WIDTH

HAS THE DELAY
TIME $t_d$ ELAPSED?          NO

YES        58

SEND SWITCH CONTROL SIGNALS TO
SWITCHES S1, S2, S3, AND S4

IS
(n) ODD OR
EVEN?

EVEN                               ODD

CLOSE SWITCHES          CLOSE SWITCHES
S2 AND S3 TO           S1 AND S4 TO
PROVIDE A              PROVIDE A
NEGATIVE PHASE   60   POSITIVE PHASE

HAS THE
PULSE WIDTH
CALCULATED IN STEP 56          NO
ELAPSED?

YES        61

OPEN THE SWITCHES WHICH WERE CLOSED
IN STEP 60

SELECT PULSE WIDTH PERCENTAGE FOR NEXT PHASE    62
AND PROVIDE TO THE MULTIPLIER

END

NO    HAS LAST
PHASE BEEN
PROVIDED?

# FIG. 4c